(19) Europäisches Patentamt
European Patent Office
Office européen des brevets

(11) Numéro de publication: **0 301 936 B1**

# (12) FASCICULE DE BREVET EUROPEEN

(45) Date de publication de fascicule du brevet: **16.12.92**

(51) Int. Cl.5: **C07D 401/12**, A61K 31/505

(21) Numéro de dépôt: **88401809.4**

(22) Date de dépôt: **12.07.88**

## (54) Dérivés de pipéridine, leur préparation et leur application en thérapeutique.

(30) Priorité: **23.07.87 FR 8710408**

(43) Date de publication de la demande:
**01.02.89 Bulletin 89/05**

(45) Mention de la délivrance du brevet:
**16.12.92 Bulletin 92/51**

(84) Etats contractants désignés:
**AT BE CH DE ES FR GB GR IT LI LU NL SE**

(56) Documents cités:

**CHEMICAL ABSTRACTS, vol. 77, 1972, page 8, résumé no. 160005y, Columbus, Ohio, US; P.K. JESTHI et al.: "Antispasmodics derived from aminopyrimidines"**

(73) Titulaire: **SYNTHELABO**
**22, Avenue Galilée**
**F-92350 Le Plessis Robinson(FR)**

(72) Inventeur: **MANOURY, Philippe**
**L'Orée de Verrières, 38, Avenue des Vaupépins**
**F-91370 Verrières le Buisson(FR)**
Inventeur: **BINET, Jean**
**12 Hameau de la Gondole**
**F-91650 Breuillet(FR)**
Inventeur: **DEWITTE, Elisabeth**
**38, Avenue d'Orgeval**
**F-95210 St. Gratien(FR)**

(74) Mandataire: **Thouret-Lemaitre, Elisabeth et al**
**SYNTHELABO Service Brevets 22, avenue Galilée**
**F-92352 LE PLESSIS ROBINSON CEDEX(FR)**

EP 0 301 936 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des dérivés de pipéridine, leur préparation et leur application en thérapeutique.

Les composés de l'invention répondent à la formule (I) donnée en annexe, dans laquelle
n est 1,2, 3 ou 4
R est un atome d'hydrogène ou d'halogène,
R′ qui est identique à R est un atome d'hydrogène ou d'halogéne,
soit $R_1$ est H ou OH et $R_2$ est H, soit $R_1$ et $R_2$ représentent ensemble une liaison,
$R_3$ est un atome d'hydrogène ou un radical $(C_{1-4})$alkyle, et $R_4$ est H ou OH.

Les sels que peuvent former les composés (I) avec des acides pharmaceutiquement acceptables font partie de l'invention. Lorsque $R_4$ est OH, les formes tautomères des composés font partie de l'invention.

Selon l'invention, on peut préparer les composés (I) selon le schéma réactionnel donné en annexe : on fait réagir un composé (II) avec un composé $X-(CH_2)n'-R_5$ dans lequel X est un groupe labile et $R_5$ est un groupe cyano (n′ est 1, 2 ou 3), un groupe phtalimido ou un groupe $(NR_3Trit)$ où $R_3$ est H ou $(C_{1-4})$-alkyle et Trit est le groupe triphénylméthyle (n′ est 2, 3 ou 4), puis on hydrogène le composé (III) obtenu en présence de nickel de Raney (dans le cas où $R_5$ est CN) ou on l'hydrolyse en présence d'hydrazine (dans le cas où $R_5$ est phtalimido) ou d'acide chlorhydrique (dans le cas où $R_5$ est $(NR_3Trit)$ et enfin on fait réagir le composé (IV) obtenu avec soit la chloro-2 pyrimidine soit la méthylthio-2 pyrimidinone-4 dans un solvant protique ou aprotique.

Les composés de départ (II) sont des pipéridines décrites dans la littérature, dans le brevet US 2804422 et le brevet belge 836394.
Les composés $X-(CH_2)n'-R_5$ sont décrits par J. D. Billimoria and K.O. Lewis. J. Chem. Soc. 1404, 1968.

Les composés (I) dans lesquels $R_1$ et $R_2$ forment une liaison peuvent également être obtenus à partir des composés (I) correspondants dans lesquels $R_1$ est OH et $R_2$ est H, par déshydratation en milieu acide fort.

Les exemples suivants illustrent l'invention.

Les analyses et les spectres IR et RMN confirment la structure des ccmposés.

Exemple 1. α,α-diphényl [(pyrimidinyl-2 amino)-2 éthyl]-1 pipéridine-4-méthanol.

1.1. [[(Hydroxydiphénylméthyl)-4 pipéridinyl-1]-2 éthyl]-2 1H,2H-isoindoledione-1,3.

On porte, à la température du reflux, pendant 5 heures, un mélange de 15 g (0,056 mole) de α,α-diphényl-pipéridine-4 méthanol, de 14,5 g (0,056 mole) de (bromo-2 éthyl)-2 1H,2H-isoindoledione-1,3 et de 6,7 g (0,063 mole) de carbonate de sodium dans 150 ml de méthylisobutylcétone.

Après évaporation du solvant on reprend le résidu avec de l'eau et du chloroforme. On lave la phase organique avec de l'eau, la sèche, la filtre et l'évapore. On obtient une huile qui cristallise après purification sur colonne de silice. On obtient un produit fondant à 166-169 °C.

1.2. (Amino-2 éthyl)-1 α,α-diphényl-pipéridine-4-méthanol.

On agite 12 h, à la température ambiante, une solution de 11,7 g (0,026 mole) du dérivé précédent dans 200 ml de méthanol contenant 1,3 ml d'hydrazine. On évapore à sec, reprend le résidu avec de l'eau et acidifie avec de l'acide chlorhydrique. On filtre l'insoluble puis extrait avec du chlorure de méthylène.
On alcalinise la phase aqueuse, extrait avec du chlorure de méthylène. On lave la phase organique avec de l'eau, sèche, filtre et évapore.
On obtient le produit blanc fondant à 164-166 °C.

1.3. α,α-diphényl [(pyrimidinyl-2 amino)-2 éthyl]-1 pipéridine-4-méthanol.

On porte au reflux pendant 20 h, 2 g ($6{,}4.10^{-3}$mole) d'(amino-2 éthyl)-1 α,α-diphényl-pipéridine-4-méthanol avec 0,75 g ($6{,}5\text{-}10^{-3}$mol) de chloro-2 pyrimidine et 0,56 g de bicarbonate de sodium dans 25 ml d'éthanol.
On évapore le mélange sous vide, reprend le résidu avec du chlorure de méthylène et de l'eau. On sèche la phase organique, filtre, évapore.
Après purification chromatograhique de l'huile résiduelle on obtient un produit solide fondant à 76 °C.

Exemple 2. [[[bis(fluoro-4 phényl)méthyl]-4 pipéridinyl-1]-2 éthyl]amino]-2 1H-pyrimidinone-4.

2.1. [bis(fluoro-4 phényl)méthyl]-4 pipéridinyl-1]-2 éthanenitrile.

On chauffe au reflux pendant 4 h, un mélange de 28,7 g (0,1 mole) de [bis(fluoro-4 phényl)méthyl]-4 pipéridine, 11,7 g (0,11 mole) de carbonate de sodium, 6,3 ml (0,1mole) de chloro-2 éthanenitrile, quelques cristaux d'iodure de sodium dans 150 ml de méthylisobutylcétone.

On évapore le mélange réactionnel, reprend le résidu avec du chlorure de méthylène et de l'eau. On sèche la phase organique, filtre, évapore.
L'huile résiduelle cristallise dans l'éther isopropylique.
(F = 114-116 °C).

2.2. [[bis(fluoro-4 phényl)méthyl]-4 pipéridinyl-1]-2 éthanamine.

On hydrogène 11,0 g ($3,4.10^{-2}$ mole) du produit précédent en solution dans 150 ml d'éthanol saturé avec de l'ammoniac à 80 °C en présence de nickel de Raney, sous une pression d'hydrogène de 60 bars.
Lorsque l'absorption d'hydrogène est terminée, on filtre le catalyseur, évapore le filtrat à sec.
On distille l'huile résiduelle. (Eb = 180 °C sous 0,05 mm de mercure).

2.3. [[[[bis(fluoro-4 phényl)méthyl]-4 pipéridinyl-1]-2 éthyl]amino]-2 1H-pyrimidinone-4.

On chauffe au reflux 48 h, sous atmosphère d'argon, 2,0 g ($1,4.10^{-2}$ mole) de S-méthyl-thiouracile, 4,6 g ($1,4.10^{-2}$ mole) de [bis(fluoro-4 phényl)méthyl]-4 pipéridinyl-1]-2 éthanamine dans 75 ml de toluéne.
On évapore le solvant, et purifie l'huile résiduelle par chromatographie sur colonne de silice.
On prépare le chlorhydrate dans l'isopropanol.
(F = 234-237 °C).

Exemple 3. [[[bis(fluoro-4 phényl)méthylène]-4 pipéridinyl-1]-2 éthyl]méthylamino]-2 1H-pyrimidinone-4.

3.1. [bis(fluoro-4 phényl)méthylène]-4 N-méthyl-pipéridine-1-éthanamine.

On chauffe au reflux 14,3 g (0,05 mole) de [bis(fluoro-4 phényl)méthylène]-4 pipéridine, 9,5 g (0,025 mole) de bromo-2 N-méthyl N-(triphénylméthyl)-éthanamine en solution dans 75 ml de toluène.
Lorsque la réaction est terminée, on refroidit le mélange et filtre le précipité. On évapore le filtrat et reprend l'huile résiduelle avec 250 ml d'HCl N. On chauffe le mélange à 50 °C pendant 1 h, laisse une nuit au repos et filtre le précipité. On extrait le filtrat avec de l'éther puis la phase aqueuse est alcalinisée avec de la soude et extraite avec du chlorure de méthylène. On lave la phase organique avec de l'eau, séche, filtre, évapore.
On obtient le produit sous forme d'huile que l'on utilise brute pour la suite de la synthèse.

3.2. [[bis(fluoro-4 phényl)méthylène]-4 pipéridinyl-1]-2 éthyl]méthylamino]-2 1H-pyrimidinone-4.

On chauffe au reflux, sous argon pendant 48 h 1 g ($2,9.10^{-3}$ mole) de l'amine précédente en solution dans 50 ml de toluène avec 0,5 g ($2,9.10^{-3}$ mole) de S-méthyl thiouracile. On évapore la solution à sec et purifie l'huile résiduelle par chromatographie sur une colonne de silice. On obtient un produit fondant à 164-165 °C.

Exemple 4. [[méthyl(pyrimidinyl-2)amino]-2 éthyl]-1 α,α-diphényl-pipéridine-4-méthanol.

4.1. [(Méthylamino-2 éthyl]-1 α,α-diphényl-pipéridine-4-méthanol.

Un mélange de 26,7 g (0,1 mole) de α,α-diphényl pipéridine-4-méthanol, 38 g (0,1 mole) de N-méthyl N-trityl bromo-2 éthanamine, 8,4 g (0,1 mole) de bicarbonate de sodium dans 400 ml de méthylisobutylcétone est chauffé au reflux pendant 48 h. Le mélange est alors filtré bouillant et le filtrat laissé au repos, on filtre le précipité obtenu et le sèche.
F = 202-205 °C.
Le précipité est repris avec 300 ml d'HCl N, on agite 4 h à température ambiante. On filtre, lave le précipité avec de l'eau.

On alcalinise le filtrat avec de la soude concentrée, extrait avec du chlorure de méthylène. On lave la phase organique avec de l'eau, sèche, filtre et évapore. On obtient un produit fondant à 168-170 ° C.

4.2. [[méthyl(pyrimidinyl-2)amino]-2 éthyl]-1 α,α-diphénylpipéridine-4-méthanol.

On porte au reflux sous atmosphère d'argon, 4,9 g (0,015 mole) de [(méthylamino)-2 éthyl]-1 α,α-diphényl-pipéridine-4-méthanol, 1,7 g (0,015 mole) de chloro-2 pyrimidine, 1,3 g de bicarbonate de sodium dans 75 ml d'éthanol. On maintient le reflux 24 h, refroidit le mélange et le filtre. On évapore le filtrat, reprend le résidu avec de l'éther, lave la phase organique avec de l'eau, sèche, filtre, évapore.
On prépare le chlorhydrate dans l'éthanol.
F = 211-212 ° C).

Exemple 5. (Diphénylméthylène)-4 N-méthyl N-(pyrimidinyl-2)-pipéridine-1-éthanamine.

Le chlorhydrate de l'exemple 4 est repris dans 100 ml d'acide chlorhydrique 6 N et le mélange est porté au reflux pendant 2 h. On refroidit la solution, la verse dans de la glace. On alcalinise avec de la soude, extrait avec du chlorure de méthylène. On lave la phase organique avec de l'eau, sèche, filtre, évapore. On obtient un produit fondant à 98-100 ° C.

Exemple 6. [[[[bis(fluoro-4 phényl)hydroxyméthyl]-4 pipéridinyl-1]-2 éthyl]méthylamino]-2 1H-pyrimidinone-4.

6.1. α,α-bis(fluoro-4 phényl) [[(méthyl)(triphénylméthyl)amino]-2 éthyl]-1 pipéridine-4-méthanol.

On prépare le produit en utilisant le procédé décrit dans 4.1. à partir de 22,0 g (0,072 mole) de α,α-bis-(fluoro-4 phényl)-pipéridine-4-méthanol, 27,6 g (0,072 mole) de bromo-2 N-méthyl N-(triphénylméthyl)-éthanamine, 6,7 g de bicarbonate de sodium et 200 ml de méthylisobutylcétone.
F = 100 ° C.

6.2. α,α-bis(fluoro-4 phényl)[(méthylamino)-2 éthyl]-1 pipéridine-4-méthanol.

L'hydrolyse est faite comme dans 4.1. à partir de 41 g (0,068 mole) du produit précédent.
F = 146-148 ° C.

6.3. [[[[bis(fluoro-4 phényl)hydroxyméthyl]-4 pipéridinyl-1]-2 éthyl]méthylamino]-2 1H-pyrimidinone-4.

Le produit est préparé comme décrit dans 3.2. à partir de 7 g (0,02 mole) du produit précédent et de 2,8 g (0,02 mole) de S-méthyl-thiouracile dans 100 ml de toluène.
F = 166-167 ° C.

Les composés préparés à titre d'exemples sont représentés dans le tableau suivant.

Tableau

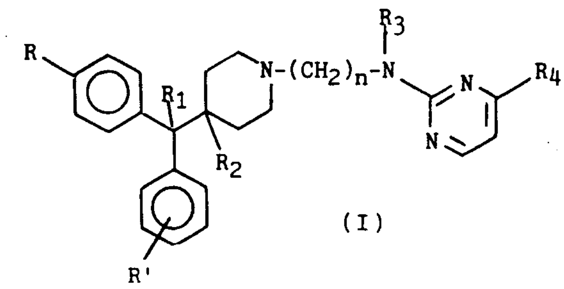

(I)

| Composé | n | R | R' | $R_1$ | $R_2$ | $R_3$ | $R_4$ | F(°C) | sel |
|---|---|---|---|---|---|---|---|---|---|
| 1 | 2 | H | H | OH | H | H | H | 76 | - |
| 2 | 2 | H | H | OH | H | H | OH | 240-243 | fumarate |
| 3 | 2 | H | H | OH | H | $CH_3$ | OH | 163-165 | - |
| 4 | 2 | H | H | OH | H | $CH_3$ | H | 211-212 | HCl |
| 5 | 2 | H | H | liaison | | $CH_3$ | OH | 177-180 | - |
| 6 | 2 | H | H | liaison | | $CH_3$ | H | 98-100 | - |
| 7 | 2 | F | 4-F | OH | H | $CH_3$ | OH | 166-167 | - |
| 8 | 2 | F | 4-F | OH | H | $CH_3$ | H | 170-173 | - |
| 9 | 2 | F | 4-F | liaison | | $CH_3$ | H | 187-189 | fumarate |
| 10 | 2 | F | 4-F | liaison | | $CH_3$ | OH | 164-165 | - |
| 11 | 2 | F | 4-F | H | H | $CH_3$ | OH | 190-192 | oxalate |
| 12 | 2 | F | 4-F | H | H | $CH_3$ | H | 222-225 | HCl |
| 13 | 2 | F | 4-F | H | H | H | H | 161-163 | fumarate |
| 14 | 2 | F | 4-F | H | H | H | OH | 234-235 | HCl |

Tableau (suite)

| Composé | n | R | R' | $R_1$ | $R_2$ | $R_3$ | $R_4$ | F(°C) | sel |
|---|---|---|---|---|---|---|---|---|---|
| 15 | 2 | F | 3-F | OH | H | $CH_3$ | OH | 123-125 | - |
| 16 | 2 | F | 3-F | liaison | | $CH_3$ | OH | 114-116 | - |
| 17 | 3 | F | 4-F | OH | H | $CH_3$ | H | 206-207 | oxalate |
| 18 | 3 | F | 4-F | liaison | | $CH_3$ | H | 171-172 | fumarate |
| 19 | 3 | F | 4-F | liaison | | $CH_3$ | OH | 211-213 | fumarate |
| 20 | 3 | F | 4-F | OH | H | $CH_3$ | OH | 144-146 | - |
| 21 | 4 | F | 4-F | liaison | | $CH_3$ | OH | 124-126 | - |

Les composés de l'invention possèdent une activité antisérotonine, [au niveau des récepteurs de type 5HT2).
Cette activité a été mise en évidence "in vitro" par déplacement de ligands fixés spécifiquement sur les récepteurs sérotoninergiques (test de binding SBS) et "in vivo" par antagonisme des effets de la sérotonine au niveau périphérique (test OES) et au niveau central (test AHT).

Test SBS : les composés de l'invention ont fait l'objet d'un essai de déplacement de la liaison (binding) du spiropéridol sur les récepteurs sérotoninergiques (5-HT2) du cortex cérébral du rat.
Pour cet essai on prélève les cerveaux de rats, on en dissèque le cortex et on l'homogénéise à 0 °C dans 10 volumes d'un mélange contenant, par litre, 50 millimoles de tampon Tris/HCl à pH = 7,4, 120 millimoles de chlorure de sodium et 5 millimoles de chlorure de potassium. On centrifuge le mélange homogène à 40000xg pendant 10 mn puis, à deux reprises, on récupère le culot, on le lave en le mettant en suspension dans le même mélange tampon, on l'homogénéise de nouveau et on le centrifuge. Pour terminer on dilue le culot final dans le même mélange tampon à raison de 100 mg de tissu humide pour 1 ml de tampon.
On soumet alors le tissu à une incubation préalable de 10 mn à 37 °C en présence de 10 micromoles/l de pargyline, puis à une incubation de 20 mn à 37 °C en présence de $^3$H-spiropéridol (activité spécifique : 25,6 Ci par millimole) à la concentration de 0,3 nanomole/l et de composé à étudier à des concentrations allant de 0,0001 à 100 micromoles/l.
On prélève des aliquots de 1 ml que l'on filtre sous vide, on lave les filtres deux fois avec 5 ml de tampon froid et on les sèche. La radioactivité est mesurée dans le toluène en présence de 5 g/l de diphényl-2,5 oxazole (PPO) et 0,1 g/l de bis-(phényl-5 oxazolyl-2)-1,4 benzène (POPOP).
Pour évaluer l'activité des composés on établit la courbe du pourcentage d'inhibition de la liaison spécifique de $^3$H-spiropéridol en fonction de la concentration en drogue déplaçante. On détermine graphiquement la concentration $IC_{50}$, concentration qui inhibe 50 % de la liaison spécifique.
La liaison spécifique est définie comme étant la liaison déplacée par 100 micromoles/l de 5-HT.
Les concentrations $IC_{50}$ des composés de l'invention se situent pour la plupart entre 1 et 50 nanomoles/l.

Test OES : l'activité antisérotoninergique des composés de l'invention a également été mise en évidence par leur effet sur l'oedème provoqué chez le rat par la sérotonine, selon la méthode décrite par Maling et coll., J. Pharmacol. Exp. Therap., 191 (2), 300-310 (1974).
Les animaux sont des rats mâles de souche CD (Ch. River, France) pesant 120 à 150 g, à jeun depuis 18 h et répartis en blocs randomisés.
Les composés, mis en solution ou en suspension dans du Tween 80$^R$ à 1%, sont administrés par voie orale

à raison de 0,5 ml pour 100 g de poids corporel, 1 h avant l'injection sous-plantaire, dans l'une des pattes postérieures, de 1 µg de sérotonine (en solution dans du sérum physiologique stérile, sous un volume de 0,1 ml). Le volume de l'oedème est mesuré 1 h après l'injection de sérotonine au moyen d'un pléthysmomètre à mercure Ugo Basile. La $DA_{40}$ (dose qui diminue de 40% le volume de l'oedème, par rapport aux animaux témoins) est déterminée graphiquement.
La $DA_{40}$ des composés de l'invention, déterminée par voie orale est comprise entre 0,1 et 2 mg/kg.

Test AHT : l'activité antisérotoninergique des composés a été étudiée quant à leur effet sur l'antagonisme des "head--twitches" (ébrouements de la tête) provoqués par le L-5-hydroxy-tryptophane (L-5-HTP) chez la souris, selon la méthode décrite par Corne et coll., Br. J. Pharmacol., 20, 106-120 (1962).
Les souris (mâles CD1, Charles River France ; 18-22 g de poids corporel) reçoivent les produits à étudier, à doses croissantes, ou le solvant, par voie intrapéritonéale ou orale, simultanément (voie i.p.) ou soixante minutes avant (voie orale) une injection de L-5-HTP à la dose de 250 mg/kg par voie sous-cutanée. Quarante-cinq minutes après cette injection de 5-HTP, le nombre d'ébrouements est compté, pour chaque souris, pendant une minute.
Pour chaque traitement, la moyenne des ébrouements, ainsi que le pourcentage de variation par rapport au lot témoin, sont calculés.
A partir de la courbe effet-dose, on détermine la $DA_{50}$ (dose active 50% ou dose qui diminue de 50% le nombre moyen d'ébrouements par rapport aux animaux témoins par la méthode graphique de Miller et Tainter (Proc. Soc. Exp. Biol. Med., (1944), 57, 261).
Les $DA_{50}$ des composés de l'invention se situent entre 0,05 et 2 mg/kg par voie intrapéritonéale et entre 0,1 et 4 mg/kg par voie orale.

Les composés de l'invention peuvent être utiles pour le traitement de la migraine, l'anxiété, la dépression, l'obésité, l'inflammation, l'asthme, les allergies, les spasmes vasculaires ou gastrointestinaux, l'hypertension et l'agrégation plaquettaire, et comme antiémétiques.
Certains composés possèdent également une activité antihistaminique.

Les composés de l'invention peuvent être administrés par voie orale ou parentérale, en association avec tout excipient approprié.

La posologie quotidienne peut aller de 5 à 200 mg.

Annexe 1

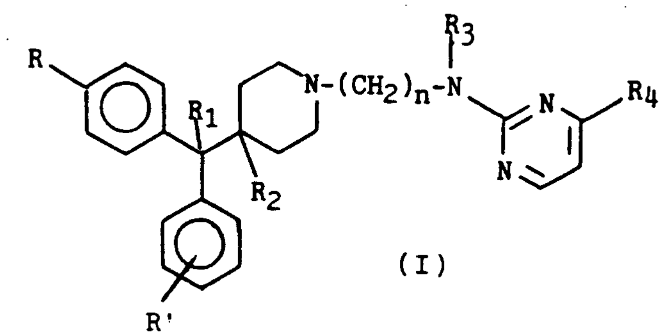

(I)

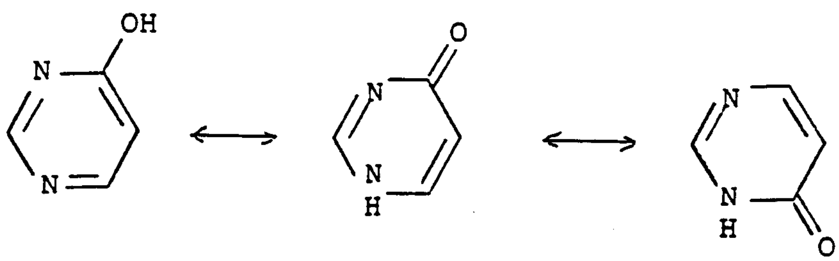

Annexe 2

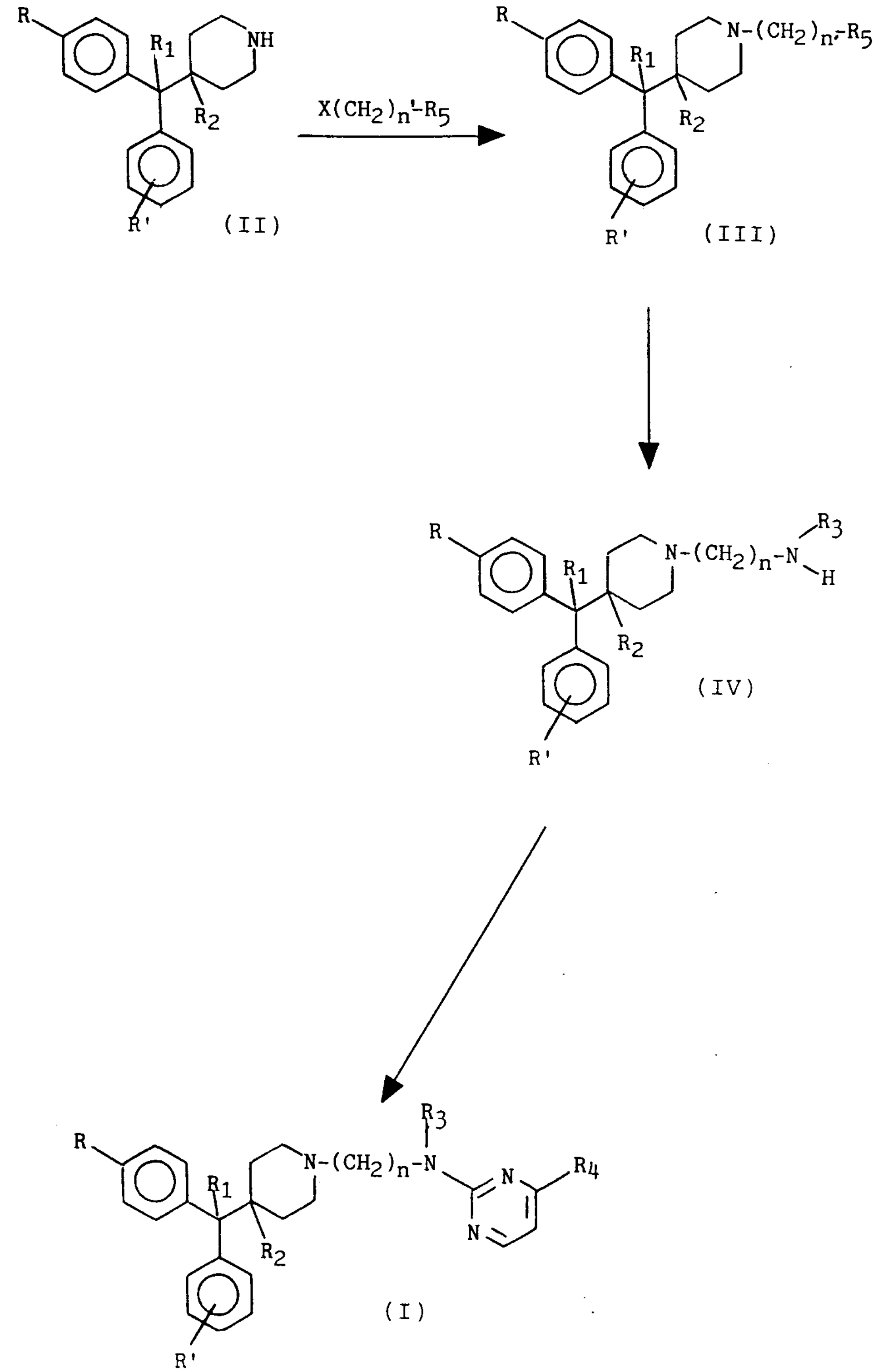

(II)
(III)
(IV)
(I)

**Revendications**

**Revendications pour les Etats contractants suivants : AT, BE, CH, DE, FR, GB, IT, LI, LU, NL, SE**

**1.** Composés répondant à la formule (I)

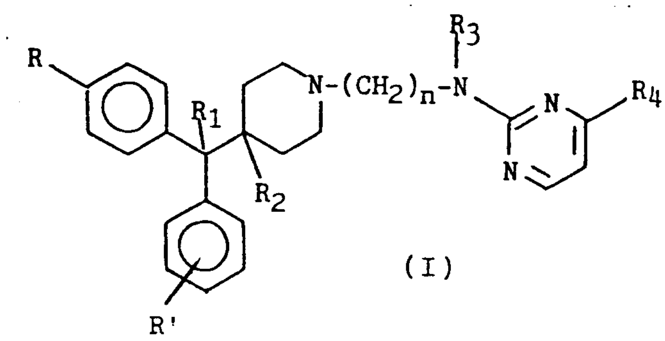

(I)

dans laquelle
n est 1,2, 3 ou 4,
R est un atome d'hydrogène ou d'halogène,
R' qui est identique à R est un atome d'hydrogène ou d'halogène,
soit $R_1$ est H ou OH et $R_2$ est H, soit $R_1$ et $R_2$ représentent ensemble une liaison,
$R_3$ est un atome d'hydrogène ou un radical $(C_{1-4})$alkyle, et
$R_4$ est H ou OH,
ainsi que les formes tautomères des composés et leurs sels d'addition aux acides pharmaceutiquement acceptables.

**2.** Procédé de préparation des composés selon la revendication 1, procédé caractérisé en ce que l'on fait réagir un composé (II)

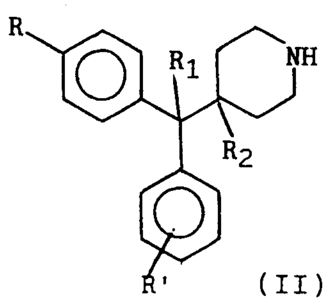

(II)

avec un composé $X-(CH_2)n'-R_5$ dans lequel X est un groupe labile et $R_5$ est un groupe cyano (n' est 1, 2 ou 3), un groupe phtalimido ou un groupe $NR_3$ Trit où $R_3$ est H ou $(C_{1-4})$alkyle et Trit est le groupe triphénylméthyle (n' est 2, 3 ou 4), puis on hydrogène le composé (III)

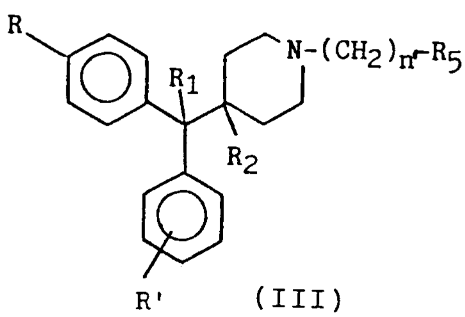

(III)

obtenu en présence de nickel de Raney (dans le cas où $R_5$ est CN) ou on l'hydrolyse en présence d'hydrazine (dans le cas où $R_5$ est phtalimido) ou d'acide chlorhydrique (dans le cas où $R_5$ est ($NR_3$Trit) et enfin on fait réagir le composé (IV)

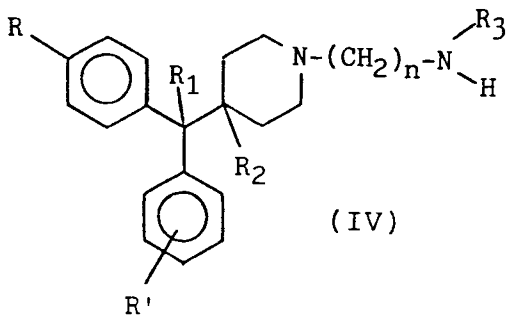

(IV)

obtenu avec soit la chloro-2 pyrimidine soit la méthylthio-2 pyrimidinone-4 dans un solvant protique ou aprotique.

3. Médicament caractérisé en ce qu'il contient un composé tel que spécifié dans la revendication 1.

4. Composition pharmaceutique caractérisée en ce qu'elle contient un composé tel que spécifié dans la revendication 1 en association avec tout excipient approprié.

**Revendication pour les Etats contractants suivants : ES, GR**

1. Procédé de préparation des composés répondant à la formule (I)

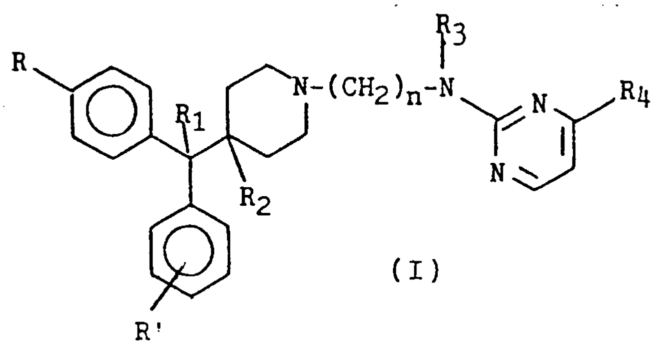

(I)

dans laquelle
n est 1,2, 3 ou 4,
R est un atome d'hydrogène ou d'halogène,
R' qui est identique à R est un atome d'hydrogène ou d'halogène,
soit $R_1$ est H ou OH et $R_2$ est H, soit $R_1$ et $R_2$ représentent ensemble une liaison,
$R_3$ est un atome d'hydrogène ou un radical $(C_{1-4})$alkyle, et
$R_4$ est H ou OH,
ainsi que de leurs formes tautomères et de leurs sels d'addition aux acides pharmaceutiquement acceptables, procédé caractérisé en ce que l'on fait réagir un composé (II)

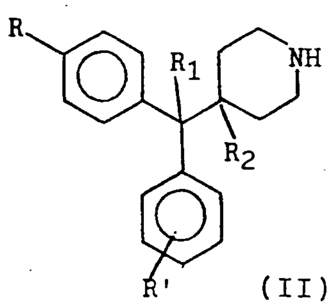

(II)

avec un composé $X-(CH_2)n'- R_5$ dans lequel X est un groupe labile et $R_5$ est un groupe cyano (n' est 1, 2 ou 3), un groupe phtalimido ou un groupe $NR_3$ Trit où $R_3$ est H ou $(C_{1-4})$alkyle et Trit est le groupe triphénylméthyle (n' est 2, 3 ou 4), puis on hydrogène le composé (III)

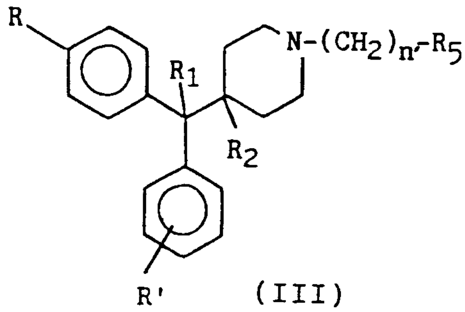

(III)

obtenu en présence de nickel de Raney (dans le cas où $R_5$ est CN) ou on l'hydrolyse en présence d'hydrazine (dans le cas où $R_5$ est phtalimido) ou d'acide chlorhydrique (dans le cas où $R_5$ est ($NR_3$Trit) et enfin on fait réagir le composé (IV)

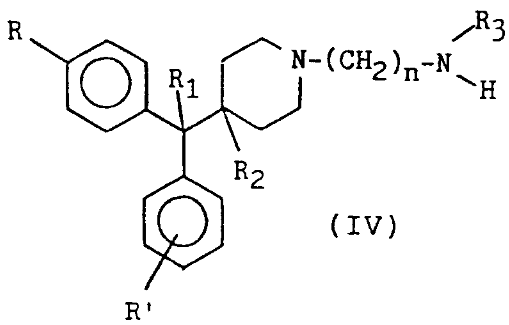

(IV)

obtenu avec soit la chloro-2 pyrimidine soit la méthylthio-2 pyrimidinone-4 dans un solvant protique ou aprotique.

## Claims

## Claims for the following Contracting States : AT, BE, CH, DE, FR, GB, IT, LI, LU, NL, SE

**1.** Compounds corresponding to the formula (I)

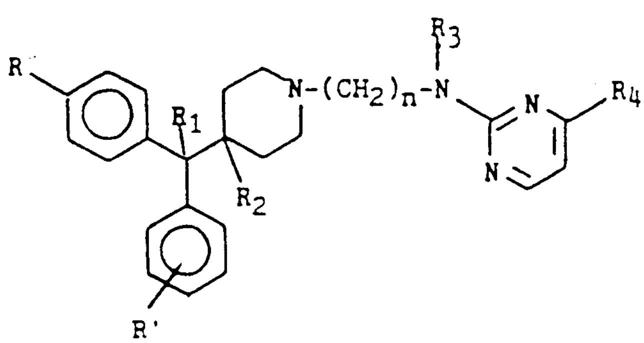

(I)

in which
n is 1, 2, 3 or 4,
R is a hydrogen or halogen atom,
R', which is identical to R, is a hydrogen or halogen atom,
either $R_1$ is H or OH and $R_2$ is H, or $R_1$ and $R_2$ together denote a bond,
$R_3$ is a hydrogen atom or a $(C_{1-4})$ alkyl radical, and

$R_4$ is H or OH,
as well as the tautomeric forms of the compounds and their addition salts with pharmaceutically acceptable acids.

**2.** Process for preparing the compounds according to Claim 1, which process is characterised in that a compound (II)

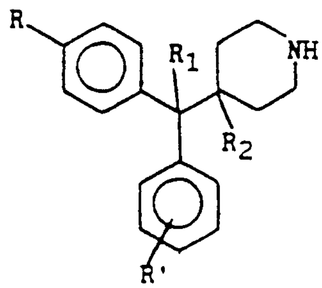

(II)

is reacted with a compound X-$(CH_2)n'$-$R_5$, in which X is a labile group and $R_5$ is a cyano group (n' is 1, 2 or 3), a phthalimido group or a group $NR_3$Trit, where $R_3$ is H or $(C_{1-4})$ alkyl and Trit is a triphenylmethyl group (n' is 2, 3 or 4), and the compound (III)

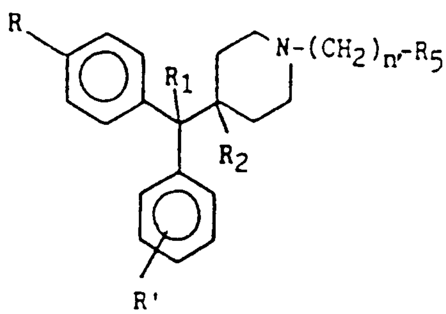

(III)

obtained is then hydrogenated in the presence of Raney nickel (in the case where $R_5$ is CN), or hydrolysed in the presence of hydrazine (in the case where $R_5$ is phthalimido) or hydrochloric acid (in the case where $R_5$ is ($NR_3$Trit)), and the compound (IV)

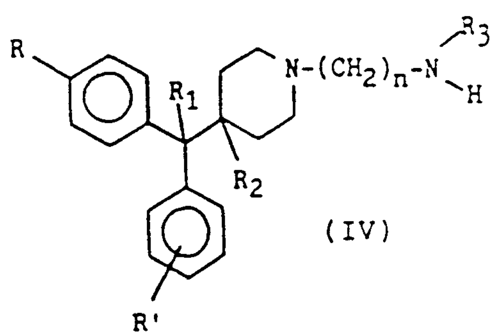

(IV)

(IV)

obtained is finally reacted with either 2-chloropyrimidine or 2-methylthio-4-pyrimidinone in a protic or aprotic solvent.

**3.** Medicinal product, characterised in that it contains a compound as specified in Claim 1.

**4.** Pharmaceutical composition, characterised in that it contains a compound as specified in Claim 1, in combination with any suitable excipient.

**Claim for the following Contracting States : ES, GR**

**1.** Process for preparing the compounds corresponding to the formula (I)

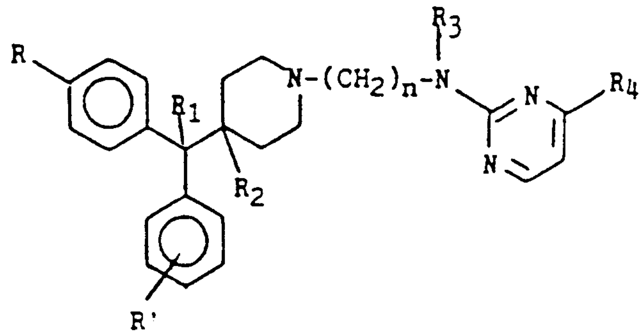

(I)

in which
n is 1, 2, 3 or 4,
R is a hydrogen or halogen atom,
R', which is identical to R, is hydrogen or halogen atom,
either $R_1$ is H or OH and $R_2$ is H, or $R_1$ and $R_2$ together denote a bond,
$R_3$ is a hydrogen atom or a $(C_{1-4})$ alkyl radical, and
$R_4$ is H or OH,
as well as their tautomeric forms and their addition salts with pharmaceutically acceptable acids, which process is characterised in that a compound (II)

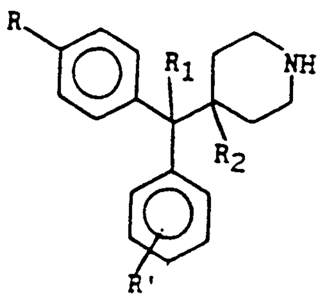

(II)

is reacted with a compound $X-(CH_2)n'-R_5$, in which X is a labile group and $R_5$ is a cyano group (n' is 1, 2 or 3), a phthalimido group or a group $NR_3$Trit, where $R_3$ is H or $(C_{1-4})$ alkyl and Trit is a triphenylmethyl group (n' is 2, 3 or 4), and the compound (III)

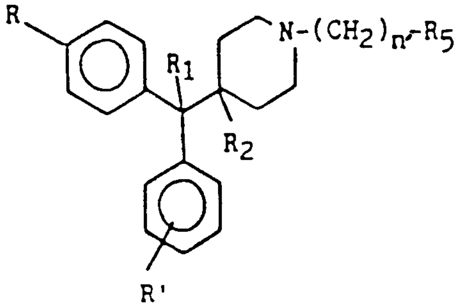

(III)

obtained is then hydrogenated in the presence of Raney nickel (in the case where $R_5$ is CN), or hydrolysed in the presence of hydrazine (in the case where $R_5$ is phthalimido) or hydrochloric acid (in the case where $R_5$ is ($NR_3$Trit)), and the compound (IV)

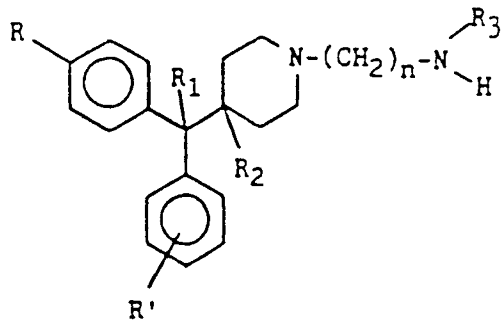

(IV)

obtained is finally reacted with either 2-chloropyrimidine or 2-methylthio-4-pyrimidinone in a protic or aprotic solvent.

**Patentansprüche**

**Patentansprüche für folgende Vertragsstaaten : AT, BE, CH, DE, FR, GB, IT, LI, LU, NL, SE**

**1.** Verbindungen der Formel (I)

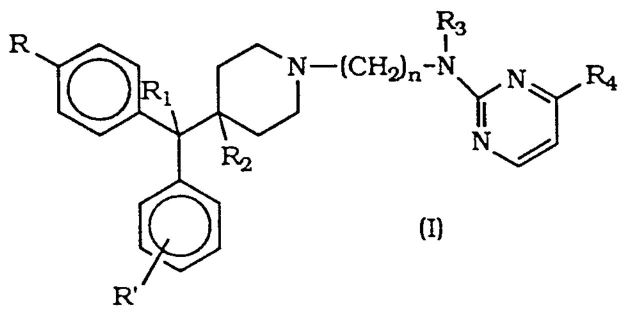

(I)

in der
n 1, 2, 3 oder 4,
R ein Wasserstoffatom oder ein Halogenatom,
R', das mit R identisch ist, ein Wasserstoffatom oder ein Halogenatom,
entweder $R_1$ H oder OH und $R_2$ H oder $R_1$ und $R_2$ gemeinsam eine Bindung,
$R_3$ ein Wasserstoffatom oder eine $(C_{1-4})$-Alkylgruppe und
$R_4$ H oder OH bedeuten,
sowie die tautomeren Formen dieser Verbindungen und deren Additionssalze mit pharmazeutisch annehmbaren Säuren.

**2.** Verfahren zur Herstellung der Verbindungen nach Anspruch 1, **dadurch gekennzeichnet,** daß man eine Verbindung der Formel (II)

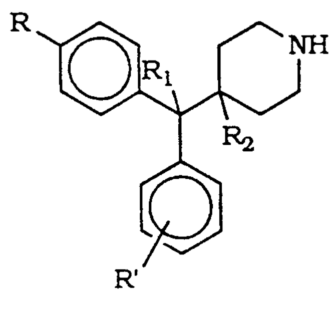

(II)

mit einer Verbindung der Formel $X-(CH_2)_{n'}-R_5$, in der X eine labile Gruppe darstellt und $R_5$ eine

Cyanogruppe (n' gleich 1, 2 oder 3), eine Phtalimidogruppe oder eine Gruppe der Formel $NR_3$ Trit, worin $R_3$ H oder eine $(C_{1-4})$-Alkylgruppe darstellt und Trit für die Triphenylmethylgruppe steht (n' gleich 2, 3 oder 4) bedeutet, umsetzt, dann die erhaltene Verbindung der Formel (III)

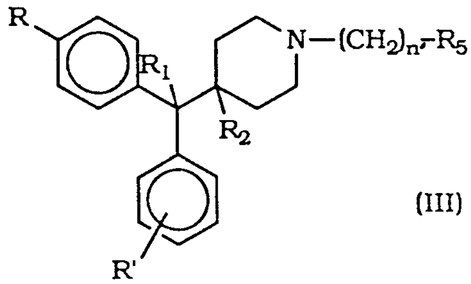

(III)

in Gegenwart von Raney-Nickel (wenn $R_5$ CN bedeutet) hydriert oder in Gegenwart von Hydrazin (wenn $R_5$ eine Phtalimidogruppe bedeutet) oder in Gegenwart von Chlorwasserstoffsäure (wenn $R_5$ eine Gruppe der Formel $NR_3$Trit darstellt) hydrolysiert und schließlich die erhaltene Verbindung der Formel (IV)

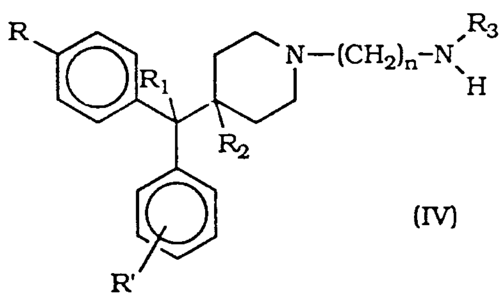

(IV)

in Gegenwart eines protischen oder aprotischen Lösungsmittels entweder mit 2-Chlor-pyrimidin oder 2-Methylthio-pyrimidin-4-on umsetzt.

3. Arzneimittel, **dadurch gekennzeichnet,** daß es eine Verbindung nach Anspruch 1 enthält.

4. Pharmazeutische Zubereitung, **dadurch gekennzeichnet,** daß sie eine Verbindung nach Anspruch 1 in Kombination mit einem geeigneten Trägermaterial enthält.

**Patentanspruch für folgende Vertragsstaaten : ES, GR**

1. Verfahren zur Herstellung der Verbindungen der Formel (I)

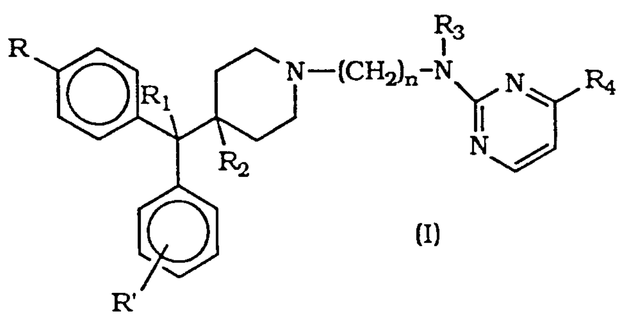

(I)

in der
n, 1, 2, 3 oder 4,

R ein Wasserstoffatom oder ein Halogenatom.
R', das mit R identisch ist, ein Wasserstoffatom oder ein Halogenatom,
entweder $R_1$ H oder OH und $R_2$ H oder $R_1$ und $R_2$ gemeinsam eine Bindung,
$R_3$ ein Wasserstoffatom oder eine $(C_1-C_4)$-Alkylgruppe und
$R_4$ H oder OH bedeuten,
sowie ihrer tautomeren Formen und ihrer Additionssalze mit pharmazeutisch annehmbaren Säuren, **dadurch gekennzeichnet,** daß man eine Verbindung der Formel (II)

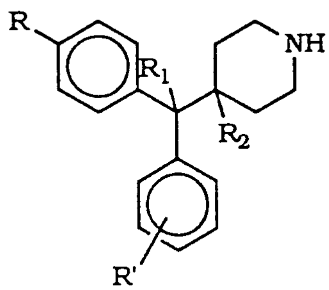

(II)

mit einer Verbindung der Formel X-$(CH_2)_{n'}$-$R_5$, in der X eine labile Gruppe darstellt und $R_5$ eine Cyanogruppe (n' gleich 1, 2 oder 3), eine Phtalimidogruppe oder eine Gruppe der Formel $NR_3$ Trit, worin $R_3$ H oder eine $(C_{1-4})$-Alkylgruppe darstellt und Trit für die Triphenylmethylgruppe steht (n' gleich 2, 3 oder 4) bedeutet, umsetzt, dann die erhaltene Verbindung der Formel (III)

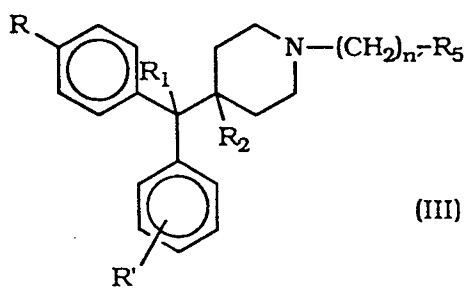

(III)

in Gegenwart von Raney-Nickel (wenn $R_5$ CN bedeutet) hydriert oder in Gegenwart von Hydrazin (wenn $R_5$ eine Phtalimidogruppe bedeutet) oder in Gegenwart von Chlorwasserstoffsäure (wenn $R_5$ eine Gruppe der Formel $NR_3$Trit darstellt) hydrolysiert und schließlich die erhaltene Verbindung der Formel (IV)

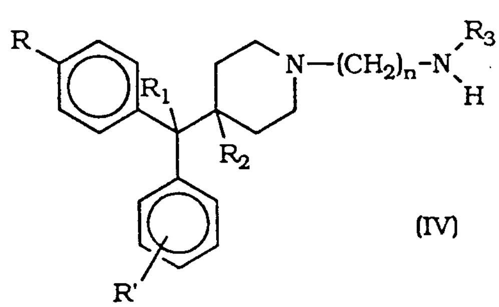

(IV)

in Gegenwart eines protischen oder aprotischen Lösungsmittels entweder mit 2-Chlor-pyrimidin oder 2-Methylthio-pyrimidin-4-on umsetzt.